# EUROPÄISCHE PATENTANMELDUNG

(11) **EP 1 046 633 A1**
(43) Veröffentlichungstag der Anmeldung: **25.10.2000**
(21) Anmeldenummer: 00107145.5
(22) Anmeldetag: 10.04.2000
(51) Int. Cl.: C07C 213/10, C07C 215/80, C07C 213/02

(54) **Verfahren zur Herstellung von Salzen von 1-substituierten 2,4-Diaminobenzolen**

(30) Priorität: 22.04.1999 DE 19918293
(71) Anmelder: Bayer Aktiengesellschaft, 51368 Leverkusen (DE)
(72) Erfinder: Rodefeld, Lars, Dr., 51371 Leverkusen (DE); Klausener, Alexander, Dr., 50259 Pulheim (DE); Behre, Horst, Dr., 51519 Odenthal (DE)

(57) **Zusammenfassung**

Bereitgestellt wird ein Verfahren zur Herstellung von speziellen Salzen von 1 -substituierten 2,4-Diaminobenzolen durch katalytische Hydrierung von 1-substituierten 2,4-Dinitrobenzolen und Zugabe des nach der Hydrierung erhaltenen und vom Katalysator befreiten Reaktionsproduktes in eine wäßrige Lösung einer Säure.

## Beschreibung

Die vorliegende Erfindung betrifft ein Verfahren zur Herstellung von Salzen von 1-substituierten 2,4-Diaminobenzolen durch katalytische Hydrierung der entsprechenden 1-substituierten 2,4-Dinitrobenzole und Überführung des erhaltenen Reaktionsproduktes nach Abtrennung des Katalysators in die Salzform.

Salze von 1-substituierten 2,4-Diaminobenzolen finden in den unterschiedlichsten Gebieten Anwendung. Ihrer Synthese kommt daher besondere Bedeutung zu. 2-(2',4'-Diaminophenoxy)-ethanol und seine Salze dienen beispielsweise gemäß DE-OS-27 58 735 und DE-OS-27 37 138 in Oxidationsfärbemittelzusammensetzungen als Meta-Komponente.

DE-OS-27 58 735 beschreibt die Herstellung von 2-(2',4'-Diaminophenoxy)-ethanol durch katalytische Hydrierung von 2-(2',4'-Dinitrophenoxy)-ethanol. Zur Abtrennung des Katalysators wird die Reaktionsmischung nach der Hydrierung im erwärmten Zustand filtriert und das Filtrat wiedergewonnen. Zur Herstellung des Dihydrochlorid-Salzes von 2-(2',4'-Diaminophenoxy)-ethanol wird anschließend ein Überschuß an Chlorwasserstoffgas in das Filtrat eingeleitet. Auf diese Weise wird jedoch nur eine Ausbeute von 78 % erhalten. Bei der Nacharbeitung dieses Verfahrens zeigt sich ferner, daß das Produkt in tiefvioletter Farbe anfällt und erhebliche Mengen an Neben- und Zersetzungsprodukten enthält, was sich in einer Reinheit von nur 86% manifestiert. Die Messung der Transmissionsgrade einer 0.1 %igen Lösung des Produktes in Wasser gemäß DIN 55945 liefert als Werte Tₓ = 38.6, T_{y}= 26.4 und T_{z}= 30.9.

Insbesondere dann, wenn die Salze der 1-substituierten 2,4-Diaminobenzole, wie 2-(2',4'-Diaminophenoxy)-ethanol, im Kosmetikbereich eingesetzt werden, z.B. als Precursor-Verbindungen auf dem Gebiet der Haarkosmetik, ist die Reinheit der 1-substituierten 2,4-Diaminobenzole ein entscheidender Faktor: Bereits geringe Kontaminationen durch Fremdstoffe sind aus medizinischen Gründen (z.B. Gefahr der Auslösung von Allergien) problematisch.

Die Aufgabe der vorliegenden Erfindung bestand somit darin, ein Verfahren bereitzustellen, mit dem Salze von 1-substituierten 2,4-Diaminobenzolen und insbesondere von 2-(2',4'-Diaminophenoxy)-ethanol mit geringen Verunreinigungen und unerwünschten Verfärbungen erhalten werden können.

Gelöst wird diese Aufgabe durch ein Verfahren zur Herstellung von Salzen von 1-substituierten 2,4-Diaminobenzolen der Formel (I) 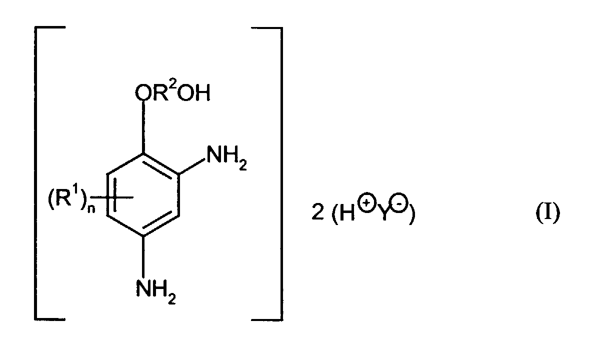
wobei R¹ unabhängig voneinander lineares oder verzweigtes C₁-C₂₀-Alkyl, C₆-C₁₈-Aryl, C₂-C₂₀-Acyl, COOH, COOR³, worin R³ für einen linearen oder verzweigten C₁-C₂₀-Alkylrest steht, oder N(R⁴)₂, worin R⁴ unabhängig voneinander für Wasserstoff, einen linearen oder verzweigten C₁-C₂₀-Alkylrest oder einen C₂-C₂₀-Acylrest stehen, bedeuten, n eine ganze Zahl von 0-3 ist
und R² einen Rest der Formel (II) 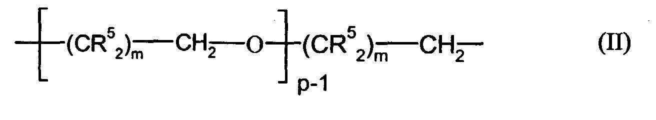
worin R⁵ unabhängig voneinander Wasserstoff oder einen C₁-C₅-Alkylrest bedeuten, m eine ganze Zahl von 1-12 und p eine ganze Zahl von 1-4 darstellt,
oder einen Rest der Formel (III) bedeutet, 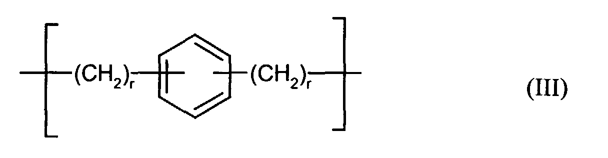
worin r unabhängig voneinander eine ganze Zahl von 0-2 ist und gegebenenfalls ein oder mehrere Kohlenstoff-Atome des Phenylrings durch N, O oder S ersetzt sind,
und Y^{⊖} das Äquivalent eines 1-3 basigen Säureanions ist,
durch katalytische Hydrierung von 1-substituierten 2,4-Dinitrobenzolen der Formel (IV), 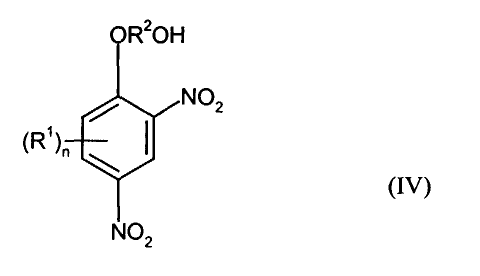
wobei R¹, n und R² die für die Formel (I) genannten Bedeutungen besitzen,
   und Zugabe des nach der Hydrierung erhaltenen und vom Katalysator befreiten Reaktionsproduktes in eine wäßrige Lösung einer Säure H^{⊕} Y^{⊖} , wobei Y^{⊖} die zuvor genannte Bedeutung besitzt.

Das erfindungsgemäße Verfahren zeichnet sich dadurch aus, daß das Produkt der Formel (I) in einer exzellenten Ausbeute von mindestens 80 %, bevorzugt mindestens 85%, besonders bevorzugt mindestens 89%, und gleichzeitig mit einer sehr hohen Reinheit von bis zu 98% sowie einer verbesserten Farbe erhalten werden kann.

In den 1-substituierten 2,4-Dinitrobenzolen der Formel (IV) bedeuten R¹ unabhängig voneinander bevorzugt lineares oder verzweigtes C₁-C₁₀-Alkyl, C₆-C₁₂-Aryl, C₂-C₆-Acyl oder N(R⁴)₂, worin R⁴ unabhängig voneinander für Wasserstoff, einen linearen oder verzweigten C₁-C₆-Alkylrest oder einen C₂-C₆-Acylrest stehen. Ferner ist n bevorzugt eine ganze Zahl von 0-2.

R² steht bevorzugt für einen Rest der Formel (II), worin m eine ganze Zahl von 1-9, insbesondere 1, 2 oder 3 ist, R⁵ Wasserstoff oder Methyl bedeutet und p gleich 1 oder 2 ist. Besonders bevorzugt steht der Rest R² für -[CH₂-CH₂]-, -[CH(CH₃)-CH₂]- oder -[CH₂-C(CH₃)₂-CH₂]-, wobei p gleich 1 ist.

R² steht ferner bevorzugt für einen Rest der Formel (III), wobei r gleich 0 oder 1 ist. Besonders bevorzugt steht der Rest R² für

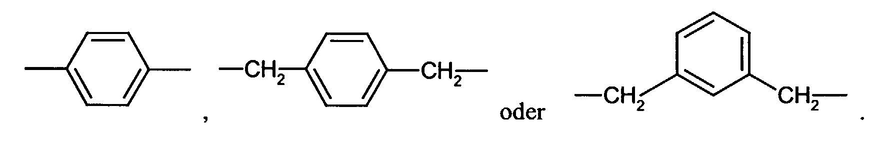

Insbesondere hat sich das erfindungsgemäße Verfahren bewährt, um aus 2-(2',4'-Dinitrophenoxy)-ethanol die Salze von 2-(2',4'-Diaminophenoxy)-ethanol herzustellen, wobei Y^{⊖} bevorzugt für Cl^{⊖}, ½ (SO₄)^{2⊖} oder 1/3 (PO₄)^{3⊖} steht.

Ein Verfahren zur Herstellung der 1-substituierten 2,4-Dinitrobenzole der Formel (IV) ist in einer am gleichen Tag eingereichten deutschen Patentanmeldung beschrieben. Weitere Verfahren sind aus DE-OS-27 58 735, J. Chem. Soc. 1921 (119), 2076-8 und US-A-2,988,571 bekannt.

Die Hydrierung der 1-substituierten 2,4-Dinitrobenzole gemäß Formel (IV) wird in Gegenwart von Hydrierkatalysatoren durchgeführt, die dem Fachmann hinlänglich bekannt sind. Hierbei kann es sich beispielsweise um Edelmetall-Katalysatoren auf der Basis von Platin oder Palladium handeln. Ferner sind auch Raney-Nickel oder Raney-Kobalt Katalysatoren verwendbar, die gegebenenfalls mit weiteren Metallen wie Molybdän, Titan, Vanadium, Magnesium, Eisen oder Chrom legiert sein können. Die Hydrierkatalysatoren können dabei geträgert oder ungeträgert sein.

Die Hydrierung wird in der Regel in Gegenwart eines Lösungsmittels durchgeführt. Als Lösungsmittel eignen sich beispielsweise verzweigte oder unverzweigte aliphatische Alkohole, bevorzugt Isopropanol, sowie gegebenenfalls substituierte aromatische Kohlenwasserstoffe wie Toluol oder Chloraromaten.

Die Hydrierung wird bei einem Wasserstoffdruck von 0.1-10 MPa, bevorzugt 0.5-5 MPa und bei einer Temperatur von 20-150°C, bevorzugt 40-120°C durchgeführt.

Das nach der Hydrierung der 1-substituierten 2,4-Dinitrobenzole der Formel (IV) erhaltene und vom Katalysator befreite Reaktionsprodukt wird in eine wäßrige Lösung einer Säure H^{⊕} Y^{⊖} eingebracht, wobei Y^{⊖} das Äquivalent eines 1-3 basigen Säureanions ist. Bei der Säure H^{⊕} Y^{⊖} handelt es sich bevorzugt um Salzsäure, Schwefelsäure oder Phosphorsäure, d.h. Y^{⊖} steht für Cl^{⊖}, ½ (SO₄)^{2⊖} oder 1/3 (PO₄)^{3⊖}. Die Säure H^{⊕} Y^{⊖} wird dabei in einer solchen Menge vorgelegt, daß das molare Verhältnis der Säure H^{⊕} Y^{⊖} zum Reaktionsprodukt der Hydrierung, d.h. dem 1-substituierten 2,4-Diaminobenzol, mindestens 2:1 beträgt.

Es hat sich ferner bewährt, das nach der Hydrierung erhaltene und vom Katalysator befreite Reaktionsprodukt in eine Vorlage zu geben, die neben der wäßrigen Lösung der Säure H^{⊕} Y^{⊖} auch noch ein Lösungsmittel enthält, welches mit der Säure H^{⊕} Y^{⊖} und Wasser mischbar ist. Bevorzugt wird als solches Lösungsmittel Isopropanol verwendet. Hierdurch läßt sich eine weitere Steigerung der Ausbeute erzielen.

Es hat sich bewährt, die Reaktion unter Einsatz eines Inertgases durchzuführen.

### Beispiel 1:

Ein 11-Autoklaven-Reaktor wird mit 120 g 2-(2',4'-Dinitrophenoxy)-ethanol, 3 g eines 1% Platin/Kohle Katalysators, der 50% Wasser enthält, und 400 ml Isopropanol befüllt. Bei einem Wasserstoffdruck von 1MPa und einer Temperatur von 100°C wird 10 h hydriert. Anschließend wird auf 20°C abgekühlt und die Reakionsmischung unter Stickstoffatmosphäre durch einen Filter in eine Vorlage aus 240 g 37%iger Salzsäure getropft, die zuvor mehrfach mit Stickstoff gespült wurde. Die erhaltene Suspension wird ca. 30 min auf Rückflußtemperatur erhitzt und anschließend auf 10°C abgekühlt. Das nach Filtration erhaltene Produkt wird einmal mit Isopropanol gewaschen. Man erhält 113,1 g 2-(2',4'-Diaminophenoxy)-ethanol (89.1% Ausbeute) in 98 %iger Reinheit. Die Messung der Transmissionsgrade einer 0.1 %igen Lösung des Produktes in Wasser gemäß DIN 55945 liefen als Werte Tₓ = 99.4, T_{y}= 98.9 und T_{z}= 98.2.

## Patentansprüche

1. Verfahren zur Herstellung von Salzen von 1-substituierten 2,4-Diamino-benzolen der Formel (I) 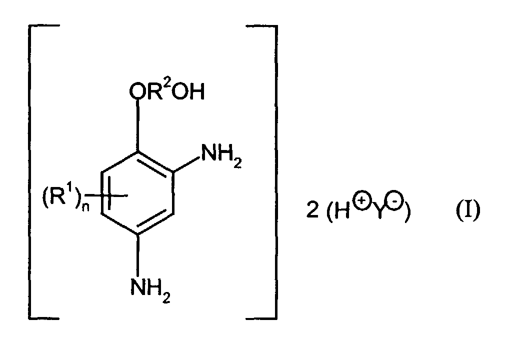
wobei R¹ unabhängig voneinander lineares oder verzweigtes C₁-C₂₀-Alkyl, C₆-C₁₈-Aryl, C₂-C₂₀-Acyl, COOH, COOR³, worin R³ für einen linearen oder verzweigten C₁-C₂₀-Alkylrest steht, oder N(R⁴)₂, worin R⁴ unabhängig voneinander für Wasserstoff, einen linearen oder verzweigten C₁-C₂₀-Alkylrest oder einen C₂-C₂₀-Acylrest stehen, bedeuten, n eine ganze Zahl von 0-3 ist,
R² einen Rest der Formel (II) 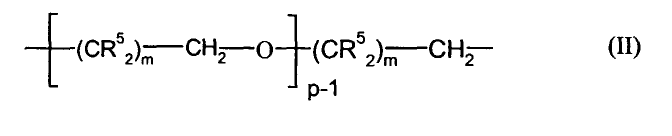
worin R⁵ unabhängig voneinander Wasserstoff oder einen C₁-C₅-Alkylrest bedeuten, m eine ganze Zahl von 1-12 und p eine ganze Zahl von 1-4 darstellt,
oder einen Rest der Formel (III) bedeutet, 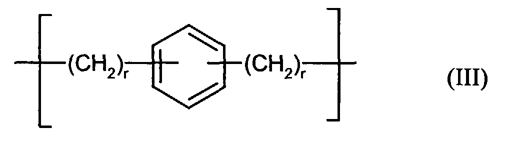
worin r unabhängig voneinander eine ganze Zahl von 0-2 ist und gegebenenfalls ein oder mehrere Kohlenstoff-Atome des Phenylrings durch N, O oder S ersetzt sind,
und Y^{⊖} das Äquivalent eines 1-3 basigen Säureanions ist,
durch katalytische Hydrierung von 1-substituierten 2,4-Dinitrobenzolen der Formel (IV), 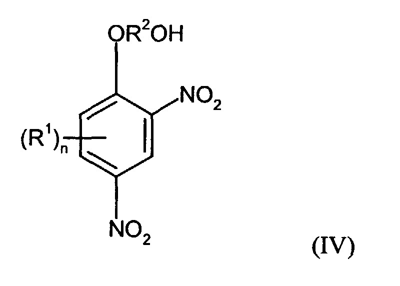
wobei R¹, n und R² die für die Formel (I) genannten Bedeutungen besitzen,
und Zugabe des nach der Hydrierung erhaltenen und vom Katalysator befreiten Reaktionsproduktes in eine wäßrige Lösung einer Säure H^{⊕} Y^{⊖}, wobei Y^{⊖} die zuvor genannte Bedeutung besitzt.

2. Verfahren nach Anspruch 1, dadurch gekennzeichnet, daß in Formel (I) R¹ unabhängig voneinander bevorzugt lineares oder verzweigtes C₁-C₁₀-Alkyl, C₆-C₁₂-Aryl, C₂-C₆-Acyl oder N(R⁴)₂, worin R⁴ unabhängig voneinander für Wasserstoff, einen linearen oder verzweigten C₁-C₆-Alkylrest oder einen C₂-C₆-Acylrest stehen, und unabhängig davon n eine ganze Zahl von 0-2 ist.

3. Verfahren nach Anspruch 1 oder 2, dadurch gekennzeichnet, daß in Formel (I) R² für einen Rest der Formel (II) steht, worin m eine ganze Zahl von 1-9 und insbesondere gleich 1, 2 oder 3 ist, R⁵ Wasserstoff oder Methyl bedeutet und p gleich 1 oder 2 ist.

4. Verfahren nach einem oder mehreren der Ansprüche 1-3, dadurch gekennzeichnet, daß der Rest R² der Formel (II) für -[CH₂-CH₂]-, -[CH(CH₃)-CH₂]- oder -[CH₂-C(CH₃)₂-CH₂]- steht, wobei p gleich 1 ist.

5. Verfahren nach Anspruch 1 oder 2, dadurch gekennzeichnet, daß R² für einen Rest der Formel (III) steht, wobei r gleich 0 oder 1 ist.

6. Verfahren nach Anspruch 5, dadurch gekennzeichnet, daß der Rest R² für 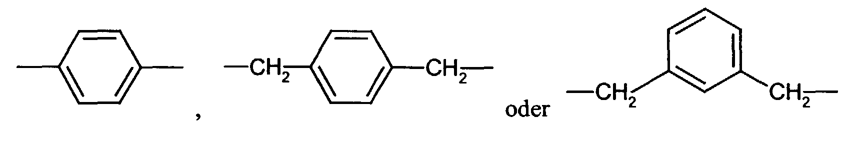 steht.

7. Verfahren nach einem oder mehreren der Ansprüche 1-6, dadurch gekennzeichnet, daß die Hydrierung der 1-substituierten 2,4-Dinitrobenzole der Formel (IV) in Gegenwart von Edelmetall-Katalysatoren auf der Basis von Platin oder Palladium oder in Gegenwart von Raney-Nickel oder Raney-Kobalt Katalysatoren durchgeführt wird.

8. Verfahren nach einem oder mehreren der Ansprüche 1-7, dadurch gekennzeichnet, daß die Hydrierung bei einem Wasserstoffdruck von 0.1-10 MPa, bevorzugt 0.5-5 MPa und bei einer Temperatur von 20-150°C, bevorzugt 40-120°C durchgeführt wird.

9. Verfahren nach einem oder mehreren der Ansprüche 1-8, dadurch gekennzeichnet, daß es sich bei der Säure H^{⊕}Y^{⊖} um Salzsäure, Schwefelsäure oder Phosphorsäure handelt.
